# EUROPÄISCHE PATENTSCHRIFT

(11) **EP 2 755 624 B1**
(45) Veröffentlichungstag und Bekanntmachung des Hinweises auf die Patenterteilung: **14.11.2018**
(21) Anmeldenummer: 12805657.9
(22) Anmeldetag: 06.12.2012
(51) Int. Cl.: A61K 6/00, A61K 6/083

(54) **DENTALMATERIALIEN AUF BASIS VON STARK ACIDEN POLYMERISIERBAREN BISPHOSPHONSÄUREN**
DENTAL MATERIAL BASED ON STRONGLY ACIDIC POLYMERISABLE BISPHOSPHONIC ACIDS
MATÉRIAUX DENTAIRES À BASE D'ACIDES BISPHOSPHONIQUES POLYMÉRISABLES TRÈS ACIDES

(30) Priorität: 06.12.2011 EP 11192246
(43) Veröffentlichungstag der Anmeldung: 23.07.2014
(73) Patentinhaber: Ivoclar Vivadent AG, 9494 Schaan (LI)
(72) Erfinder: MOSZNER, Norbert, 9495 Triesen (LI); CATEL, Yohann, 9470 Buchs SG (CH); ANGERMANN, Jörg, CH-7320 Sargans (CH); BOCK, Thorsten, A-6806 Tosters (AT); RHEINBERGER, Volker, FL-9490 Vaduz (LI)
(74) Vertreter: Uexküll & Stolberg
(86) Internationale Anmeldenummer: PCT/EP2012/074715
(87) Internationale Veröffentlichungsnummer: WO 2013/083734

(56) Entgegenhaltungen:
- EP-A1- 0 909 761
- JP-A- 2006 298 771
- US-A1- 2004 206 932

## Beschreibung

Die vorliegende Erfindung betrifft polymerisierbare Bisphosphonsäuren und deren Verwendung als Monomerkomponente in adhäsiven Dentalmaterialien und insbesondere zur Herstellung von dentalen Adhäsiven, Zementen und Kompositen.

Polymerisierbare Phosphonsäuren sind vor allem als Comonomere von polymerchemischer und technischer Bedeutung und gestatten die Herstellung von organischen Polymeren mit besserer thermischer Stabilität, verbesserten Hafteigenschaften, verminderter Entflammbarkeit und verbesserter Löslichkeit in polaren Lösungsmitteln. In diesem Zusammenhang sind zahlreiche monomere Phosphonsäuren mit polymerisierbaren Vinyl-, Dienyl-, Allyl- oder Styrylgruppen synthetisiert und polymerisiert worden (vgl. Houben-Weyl, Methoden der Organischen Chemie, Band E 20 (2.Teil), G. Thieme Verlag, Stuttgart-New York 1987, 1300 ff.). Polymerisierbare Phosphonsäuren sind auch als Komponente von Dentaladhäsiven bekannt (vgl. N. Moszner, U. Salz, J. Zimmermann, Dental Materials 21 (2005) 895-910) und Gegenstand zahlreicher Patente, wie z.B. DE 100 18 968 C1, DE 102 34 326 B3, DE 199 18 974 A1, EP 1 057 468 A1 und EP 1 169 996 A1.

Bisphosphonsäuren (BPA), bei denen zwei Phosphonat-Gruppen über eine Methylengruppe verbunden sind, finden seit vielen Jahren als diagnostische oder therapeutische Medikamente bei Knochen- oder Calciumstoffwechsel-Erkrankungen Anwendung. Beispielsweise wird Alendronsäure zur Behandlung der Osteoporose eingesetzt. Dabei ist schon seit langem bekannt (vgl. M.D. Francis et al., Science 165 (1969) 1264-1266), dass Bisphosphonate die Bildung von Calciumphosphat inhibieren, so dass Bisphosphonate in Zahnpasten zur Reduktion der subgingivalen Zahnsteinbildung eingesetzt wurden.

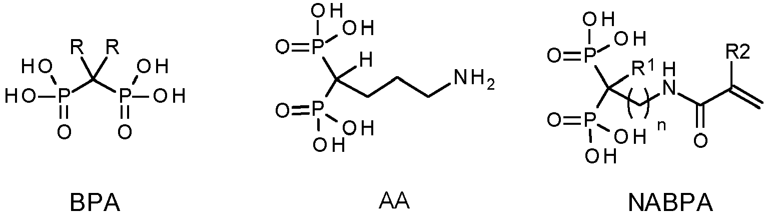

Polymerisierbare N-Acryl-aminoalkylbisphosphonsäuren (NABPA) sind in der DD 273 846 A1 als Komponente von Haftvermittlern beschrieben. Analoge Methylenbisphosphonsäure(meth)acrylamide werden in WO 2004/060327 A1 und US 2004/206932 A1 als Komponente für selbstätzende Primer beschrieben. Solche Methylenbisphosphonsäuren sind meist Feststoffe, die sich zwar in Wasser ausgezeichnet lösen, jedoch in organischen Lösungsmitteln kaum löslich sind. Methylenbisphosphonsäureacrylamide mit verbesserter Löslichkeit wurden für dentale Adhäsive von Y. Catel, M. Degrange, L. L. Pluart, P.-J. Madec, T.-N. Phan, F. Chen, W. D. Cook (J. Polym. Sci.:Part A: Polym. Chem. 47 (2009) 5258-5271) synthetisiert. WO 03/013444 A1 beschreibt selbstätzende Primer auf Basis von Bisphosphonsäuren mit zwei polymerisierbaren (Meth)acrylamidgruppen, wobei die Phosphonsäuregruppen jeweils direkt oder über Spacer an die Stickstoffatome der (Meth)acrylamidgruppen gebunden und diese Stickstoffatome ihrerseits über einen weiteren Spacer miteinander verknüpft sind. Diese Verbindungen zeichnen sich durch eine verminderte radikalische Polymerisierbarkeit aus. Schließlich sind einige aromatische Bisphosphonsäuren bekannt, die zwei polymerisierbare Methacrylgruppen tragen, z.B. BPA-1 (N. Moszner, Macromol. Chem. Phys. 200 (1999) 1062-1067; EP 0 909 761 A1) und BPA-2 (L. Mou, G. Singh, J. W. Nicholson, Chem. Commun. 2000, 345-346; N. Sibold, P.-J. Madec, S. Masson, T.-N. Phan, Polymer 43 (2002) 7257-7267; G. Sahin, A. Z. Albayrak, Z. S. Bilgici, D. Avci, J. Polym. Sci.:Part A: Polym. Chem. 47 (2009) 1953-1965).

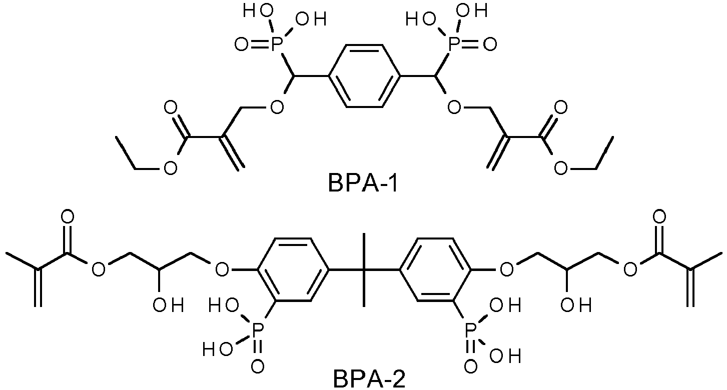

Solche aromatischen Bisphosphonsäure-Derivate zeichnen sich aber insgesamt durch eine geringe Löslichkeit in Wasser, Aceton, Alkohol und Mischungen davon aus.

JP2006298771A beschreibt organische Phosphorverbindungen mit guter Haftfähigkeit, in denen polymerisierbare Gruppen über Spacer an eine Bisphosphorsäureeinheit gebunden sind.

Der Erfindung liegt daher die Aufgabe zugrunde, adhäsive Dentalwerkstoffe bereitzustellen, die gut polymerisierbar, stark acide und in polaren Lösungsmitteln und deren Mischungen mit Wasser löslich sind, eine gute Substrathaftung zur Zahnhartsubstanz und/oder Dentalkeramiken vermitteln und sich damit vor allem zur Herstellung von Adhäsiven, adhäsiven Zementen und Kompositen eignen.

Die Aufgabe wird erfindungsgemäß gelöst durch Dentalwerkstoffe auf Basis einer polymerisierbaren Bisphosphonsäure der Formel III: 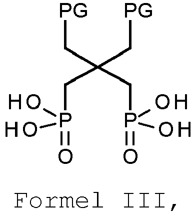 in der
- PG: unabhängig für eine polymerisierbare Gruppe ausgewählt aus CH₂=CR²-CO-O- und CH₂=CR²-CO-NR³- und insbesondere für CH₂=CR²-CO-O- steht,
- R²: unabhängig für H oder CH₃ und vorzugsweise für CH₃ steht und
- R³: unabhängig für H oder vorzugsweise für einen C₁-C₁₀-Alkyl-Rest, insbesondere für einen C₁-C₇-Rest, bevorzugt für einen C₁-C₅-Rest, weiter bevorzugt für einen C₁-C₃-Rest und am meisten bevorzugt für CH₃ oder C₂H₅ steht,
oder der Formel V: 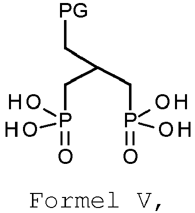 in der
- PG: für eine polymerisierbare Gruppe ausgewählt aus CH₂=CR²-CO-O-, CH₂=CR²-CO-NR³- und R⁴O-CO-C(=CH₂)-CH₂-O-, insbesondere ausgewählt aus CH₂=CR²-CO-O- und CH₂=CR²-CO-NR³- und bevorzugt für CH₂=CR²-CO-NR³- steht,
- R²: für H oder CH₃ und vorzugsweise für CH₃ steht,
- R³: für H oder vorzugsweise für einen C₁-C₁₀-Alkyl-Rest, insbesondere für einen C₁-C₇-Rest, bevorzugt für einen C₁-C₅-Rest, weiter bevorzugt für einen C₁-C₃-Rest und am meisten bevorzugt für CH₃ oder C₂H₅ steht und
- R⁴: für H, einen C₁-C₁₀-Alkyl-Rest, Phenyl oder Mesityl steht.

Dabei ist es weiter bevorzugt, dass in der Formel III die Gruppen PG, R² bzw. R³ jeweils gleich sind.

Erfindungsgemäß werden nur solche Verbindungen in Erwägung gezogen, die mit der chemischen Valenzlehre vereinbar sind.

Es wurde überraschend gefunden, dass die erfindungsgemäßen Dentalwerkstoffe, die mindestens eine polymerisierbare Bisphosphonsäure der Formel III oder V enthalten, eine sehr hohe radikalische Polymerisationsneigung zeigen und zugleich stark acide und gut löslich in polaren Lösungsmitteln und deren Mischungen mit Wasser sind und nach der Polymerisation eine hervorragende Haftung auf Zahnhartsubstanz und Dentalkeramiken zeigen.

Die polymerisierbaren Bisphosphonsäuren lassen sich einfach herstellen. So kann die Einführung der Phosphonatgruppen beispielsweise durch eine Michaelis-Arbuzov-Reaktion ausgehend von mindestens zweifach unterschiedlich funktionalisierten Verbindungen erfolgen, die zwei phosphonierbare Gruppen Y wie z.B. Halogen enthalten (vgl. Autorenkollektiv, Organikum, 21. Aufl., Wiley-VCH, Weinheim etc., 2001, 246 ff.), wobei die vorhandenen weiteren funktionellen Gruppen D, wie z.B. OH oder NHR³, gegebenenfalls durch entsprechende Schutzgruppen zu schützen sind. In das erhaltene Bisphosphonat können dann durch Umsetzung mit einem Acrylsäurederivat CH₂=C(R²)-CO-Z die polymerisierbaren Reste eingeführt und anschließend die Phosphonsäuregruppen durch Umsetzung mit Trimethylsilylbromid (TMSBr) und anschließender Methanolyse (vgl. S. Freeman, J. Chem. Soc., Perkin Trans. 2 (1991) 263.) freigesetzt werden:

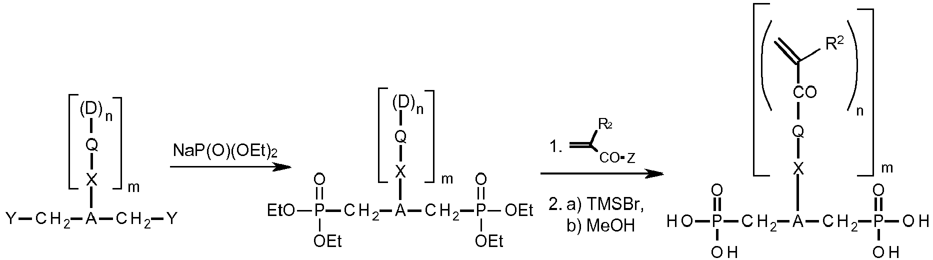

Konkretes Beispiel: Ausgehend von käuflichem 2,2-(Bis(brommethyl)-1,3-propandiol (A=CH, X entfällt, Q=CH₂, D=OH, Y= Br, m = 2, n = 1) werden die beiden OH-Gruppen durch Ketalisierung z.B. mit Aceton geschützt. Dann erfolgt die Einführung der beiden Phosphonsäuregruppen durch Umsetzung mit Natriumdiethylphosphit. Aus dem gebildeten Bisphosphonat werden die Schutzgruppen durch Methanolyse in Gegenwart eines stark sauren Ionenaustauscherharzes als Katalysator abgespalten, die polymerisationfähigen Methacrylatgruppen (PG = CH₂=C(CH₃)-CO-O-) durch Acylierung mit Methacrylsäurechlorid (R² = CH₃, Z = Cl) eingeführt und schließlich die Phosphonsäuregruppen freigesetzt:

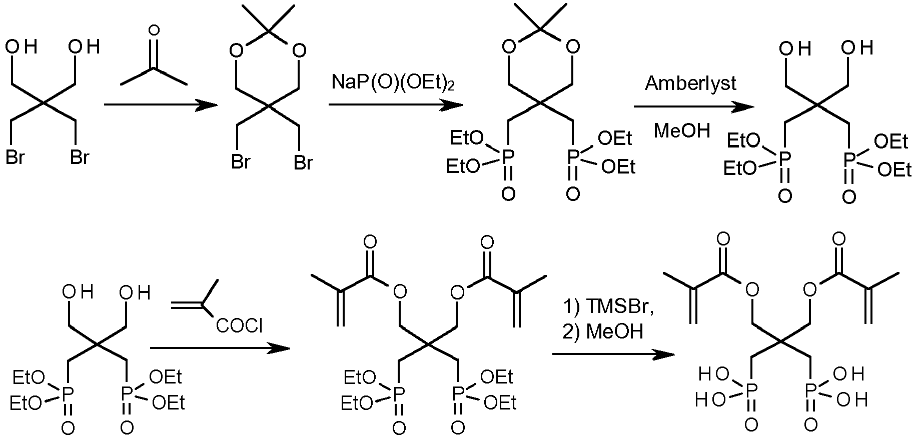

Geeignete Ausgangsstoffe für die Synthese von polymerisierbaren Bisphosphonsäuren sind neben Pentaerythrit auch andere kommerzielle mehrwertige Alkohole wie z.B. Glycerin, Trimethylolethan, 1,2,4-Butantriol oder Trimethylolpropan.

Eine andere prinzipielle Synthesemöglichkeit für polymerisierbare Bisphosphonsäuren besteht darin, einen Bisphosphonat-Baustein und einen polymerisierbaren Baustein getrennt zu synthetisieren und danach beide Bausteine zu verbinden.

Beispiele für erfindungsgemäße polymerisierbare Bisphosphonsäuren der Formel III oder V sind:

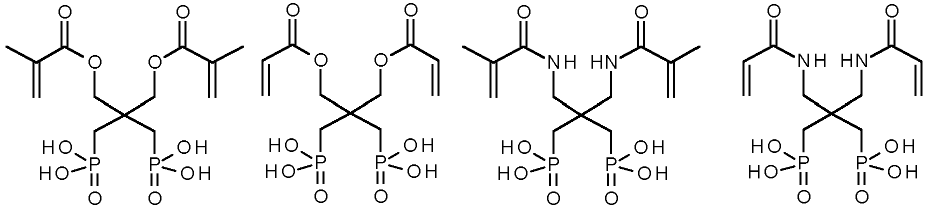

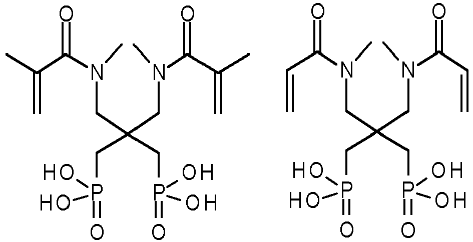

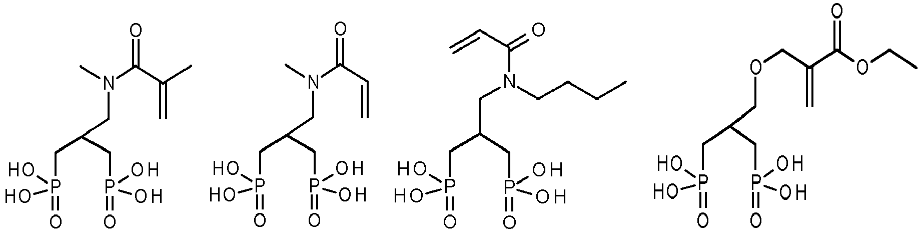

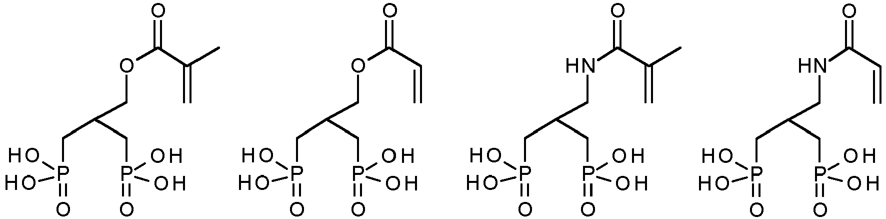

Die erfindungsgemäßen Dentalmaterialien enthalten neben der polymerisierbaren Bisphosphonsäure der Formel III oder V vorzugsweise ein oder mehrere zusätzliche radikalisch polymerisierbare Monomere (Co-Monomere), insbesondere mono- oder polyfunktionelle (Meth)acrylsäurederivate. Unter monofunktionellen (Meth)acrylsäurederivaten werden Verbindungen mit einer, unter polyfunktionellen (Meth)acrylsäurederivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäß bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi(meth)acrylat, 1,4-Butandioldi(meth)acrylat, 1,10-Decandioldi(meth)acrylat und 1,12-Dodecandioldi(meth)acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis(3-vinyl-2-pyrrolidonyl)-hexan, Bisacrylamide wie Methylen- oder Ethylenbisacrylamid und Bis(meth)acrylamide wie N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können.

Vorzugsweise werden Mischungen der vorstehend genannten Monomere verwendet.

Die erfindungsgemäßen Dentalwerkstoffe können neben der polymerisierbaren Bisphosphonsäure der Formel III oder V und ggf. den oben genannten Co-Monomeren vorzugweise auch zusätzliche radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisierbaren Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Monomere mit polymerisierbaren Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester.

Bevorzugte Monomere mit polymerisierbaren Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte Monomere mit polymerisierbaren Sulfonsäuregruppen sind Vinylsulfonsäure, 4-Vinylphenylsulfonsäure und 3-(Meth-acrylamido)propylsulfonsäure.

Außerdem enthalten die erfindungsgemäßen Dentalwerkstoffe vorzugsweise auch einen Initiator für die radikalische Polymerisation.

Zur Initiierung der radikalischen Photopolymerisation werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4'-Dichlorbenzil eingesetzt. Besonders bevorzugt werden Campherchinon und 2,2-Dimethoxy-2-phenyl-acetophenon und ganz besonders bevorzugt α-Diketone in Kombination mit Aminen wie 4-(Dimethylamino)-benzoesäureester, N,N-Dimethylaminoethylmethacrylat, N,N-Dimethyl-sym.-xylidin oder Triethanolamin als Reduktionsmittel verwendet. Besonders geeignet sind auch Norrish-Typ-I-Photoinitiatoren, insbesondere Acyl- der Bisacylphosphinoxide, Monoacyltrialkyl- oder Diacyldialkylgermanium-Verbindungen wie Benzoyltrimethylgermanium, Dibenzoyldiethylgermanium oder Bis-(4-methoxybenzoyl)diethylgermanium. Dabei lassen sich auch Mischungen der verschiedenen Photoinitiatoren wie beispielsweise Dibenzoyldiethylgermanium in Kombination mit Campherchinon und 4-Dimethylaminobenzoesäureethylester einsetzen.

Als Initiatoren für eine bei Raumtemperatur durchgeführte Polymerisation werden vorzugsweise Redox-Initiatorkombinationen wie beispielsweise Kombinationen von Benzoylperoxid mit N,N-Dimethyl-sym.-xylidin oder N,N-Dimethyl-p-toluidin verwendet. Darüber hinaus sind auch Redoxsysteme bestehend aus Peroxiden und solchen Reduktionsmitteln wie z.B. Ascorbinsäure, Barbituraten oder Sulfinsäuren besonders geeignet.

Weiterhin enthalten die erfindungsgemäß eingesetzten Zusammensetzungen zur Verbesserung der mechanischen Eigenschaften oder zur Einstellung der Viskosität vorzugsweise auch organische oder anorganische Füllstoffpartikel. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgröße von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgröße von 5 bis 200 nm, vorzugsweise 10 bis 100 nm, Minifüllstoffe wie Quarz-, Glaskeramik- oder Glaspulver mit einer durchschnittlichen Teilchengröße von 0,01 bis 10 µm, vorzugsweise 0,1 bis 1 µm, sowie röntgenopake Füllstoffe wie Ytterbiumtrifluorid oder nanopartikuläres Tantal(V)-oxid bzw. Bariumsulfat mit einer mittleren durchschnittlichen Partikelgröße von 10 bis 1000 nm, vorzugsweise 100 bis 300 nm.

Außerdem können die erfindungsgemäß eingesetzten Zusammensetzungen weitere Additive enthalten, vor allem Lösungsmittel wie Wasser oder Ethanol bzw. entsprechende Lösungsmittelgemische, sowie beispielsweise Stabilisatoren, Aromastoffe, Farbmittel, mikrobiozide Wirkstoffe, fluoridionenabgebende Additive, optische Aufheller, Weichmacher oder UV-Absorber.

Besonders bevorzugt sind Dentalwerkstoffe auf Basis einer polymerisierbaren Bisphosphonsäure der Formel III oder V, welche die folgenden Bestandteile enthalten:
a) 0,1 bis 50 Gew.-%, insbesondere 1 bis 40 Gew.-%, bevorzugt 2 bis 30 Gew.-% und besonders bevorzugt 5 bis 20 Gew.-% polymerisierbare Bisphosphonsäure der Formel III oder V,
b) 0,01 bis 10 Gew.-%, bevorzugt 0,1 bis 3 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff und
f) 0 bis 70 Gew.-%, bevorzugt 0 bis 60 Gew.-% und besonders bevorzugt 0 bis 50 Gew.-% Lösungsmittel.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff.

Dies gilt ebenso für den Lösungsmittelgehalt. Adhäsive enthalten vorzugweise bevorzugt 0 bis 60 Gew.-% und besonders bevorzugt 1 bis 50 Gew.-% Lösungsmittel. Dentalmaterialien, die Wasser als Lösungsmittel enthalten, sind bevorzugt. Besonders bevorzugt sind Dentalmaterialien, die 0 bis 20 Gew.-% und insbesondere 1 bis 10 Gew.-% Wasser enthalten.

Die Erfindung wird im Folgenden anhand von Ausführungsbeispielen näher erläutert.

### Ausführungsbeispiele

### Beispiel 1

### Synthese von 3-(Methacryloyloxy)-2,2-(di[(dihydroxyphosphoryl)-methyl])propylmethacrylat (DMPBPA)

### 1. Stufe: 5,5-Di[(diethoxyphosphoryl)methyl]-2,2-dimethyl-1,3-dioxan

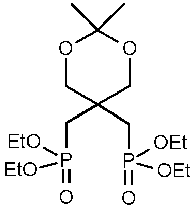

Natriumdiethylphosphit (63,6 g, 400 mmol, 6,0 äq.) wurde zu einer Lösung von 5,5-Di(brommethyl)-2,2-dimethyl-1,3-dioxan (20,0 g, 66 mmol) in einer wasserfreiem Mischung von THF/DMF (9:1, 600 ml) gegeben. Die Reaktionsmischung wurde 15 h bei 68 °C gerührt, anschließend mit einer gesättigten wässrigen Ammoniumchlorid-Lösung (20,0 ml) versetzt und die flüchtigen Bestandteile im Vakuum abgezogen. Der Rückstand wurde mit entionisiertem Wasser (200 ml) verdünnt und die gebildete Lösung mit Methylenchlorid (3 x 200 ml) extrahiert. Die vereinigten organischen Phasen wurden mit wasserfreiem Natriumsulfat getrocknet und anschließend das Lösungsmittel unter vermindertem Druck entfernt. Das erhaltene Rohprodukt durch Flash-Säulenchromatographie (Eluent: Ethylacetat/Methanol 9:1) gereinigt. Es wurden 19,6 g (71% Ausbeute) des Produkts als farbloses Öl erhalten.

¹H-NMR (400 MHz, CDCl₃) : δ = 1,32 (t, ³J_{HH} = 7,0 Hz, 12H, POCH₂CH₃) ; 1,41 (s, 6H, CH₃); 2,29 (d, ²J_{HP} = 19,2 Hz, 4H, CH₂P); 3,85 (s, 4H, CCH₂O) ; 4,05-4,15 (m, 8H, POCH₂CH₃), ³¹P NMR (162 MHz, CDCl₃) : δ = 29,0.
¹³C-NMR (101 MHz, CDCl₃) : δ = 16,4 (d, ³J_{CP} = 6,5 Hz, POCH₂CH₃) ; 23,8 (CH₃); 28,3 (dd, ¹J_{CP} = 138,3 Hz, ³J_{CP} = 4,0 Hz, CH₂P); 33,7 (t, ²J_{CP} = 3,0 Hz, CCH₂P) ; 61,6 (d, ²J_{CP} = 6,7 Hz, POCH₂CH₃); 68,0 (t, ³J_{CP} = 10,7 Hz, OCH₂CCH₂P) ; 98,3 (CCH₃).
HRMS (*m*/*z*): Ber. für C₁₆H₃₅O₈P₂: 417,18; gef.: 417,18 [M + H]⁺.

### 2. Stufe: 3-(Methacryloyloxy)-2,2-(di[(diethoxyphosphoryl)methyl])-propylmethacrylat

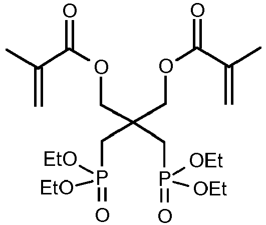

Zu einer Lösung des Bisphosphonats aus der 1. Stufe (19,55 g, 47 mmol) in wasserfreiem Methanol (600 ml) wurden 1.95 g des Ionenaustauscherharzes Amberlyst H-15 gegeben, und die Mischung wurde 11 h bei 25 °C gerührt. Nach Filtration der Lösung wurde das Lösungsmittel im Vakuum abgezogen. Es wurden 17,5 g eines öligen Rohproduktes erhalten, die zu einer Mischung aus trockenem Methylenchlorid (120 ml), Triethylamin (16,9 ml, 12 mmol) und 4-(N,N-Dimethylamino)-pyridin (489 mg, 4,0 mmol) gegeben wurden. Anschließend wurde Methacrylsäureanhydrid (18,1 ml, 12 mmol) bei Raumtemperatur langsam zugetropft und die Reaktionsmischung 15 h gerührt. Zur Aufarbeitung wurden entionisiertes Wasser (60 ml) zugegeben, die Phasen getrennt und die wässrige Phase mit Methylenchlorid (3 x 60 ml) extrahiert. Die vereinigten organischen Phasen wurden mit wasserfreiem Natriumsulfat getrocknet. Nach Abtrennung des Lösungsmittels unter vermindertem Druck wurde das erhaltene Rohprodukt durch Flash-Säulenchromatographie (Eluent: Ethylacetat/ Methanol 97:3) gereinigt. Es wurden 10,3 g (43% Ausbeute) des Produkts als gelber Feststoff (Fp. = 43-45 °C) erhalten.

¹H-NMR (400 MHz, CDCl₃) : δ = 1,28 (t, ³J_{HH} = 7,0 Hz, 12H, POCH₂CH₃) ; 1,93 (s, 6H, CH₂=CCH₃) ; 2,34 (d, ²J_{HP} = 19,9 Hz, 4H, CH₂P); 4,03-4,14 (m, 8H, POCH₂CH₃) ; 4,22 (s, 4H, CCH₂O) ; 5,55-5,58 (m, 2H, C=CH₂); 6,08-6,11 (m, 2H, C=CH₂),
³¹P-NMR (162 MHz, CDCl₃): δ = 28,0.
¹³C-NMR (101 MHz, CDCl₃) : δ = 16,3 (d, ³J_{CP} = 6,2 Hz, POCH₂CH₃); 18,3 (CH₂=CCH₃); 27,8 (dd, ¹J_{CP} = 139,5 Hz, ³J_{CP} = 4,3 Hz, CH₂P); 38,3 (t, ²J_{CP} = 2,7 Hz, CCH₂P); 61,7 (d, ²J_{CP} = 7,0 Hz, POCH₂CH₃); 66,5 (t, ³J_{CP} = 12,8 Hz, OCH₂CCH₂P); 126,0 (C=CH₂); 136,0 (C=CH₂); 166,6 (C=O).
HRMS (*m*/*z*): Ber. für C₂₁H₃₉O₁₀P₂: 513,20; gef.: 513,20 [M + H]⁺.

### 3. Stufe: 3-(Methacryloyloxy)-2,2-(di[(dihydroxyphosphoryl)methyl])-propylmethacrylat (DMPBPA)

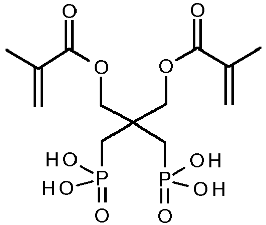

Zu einer Lösung des Bisphosphonats aus der 2. Stufe (9,6 g, 18,75 mmol) in Methylenchlorid (80 ml) wurde Trimethylsilylbromid (14,9 ml, 112,5 mmol, 6 äq.) zugetropft und die Reaktionsmischung 5 h bei 30 °C gerührt. Anschließend wurde die Mischung unter vermindertem Druck eingeengt, mit Methanol (80 ml) versetzt und 30 min bei Raumtemperatur gerührt. Nach Zugabe von 3,5-Di-tert-butyl-4-hydroxytoluol (BHT, 200 ppm) und Galvinoxyl-Radikalen (20 ppm) wurden flüchtige Bestandteile im schwachen Vakuum entfernt und das Produkt im Feinvakuum (0,07 mbar) bis zur Gewichtskonstanz getrocknet. Es wurden 7,1 g (95% Ausbeute) des Produkts als weißer Feststoff (Fp. > 142 °C) mit sehr guter Löslichkeit in Wasser, Ethanol, Aceton und THF erhalten.

¹H-NMR (400 MHz, MeOH-d₄): δ = 1,95 (s, 6H, CH₂=CCH₃); 2,26 (d, ²J_{HP} = 19,6 Hz, 4H, CH₂P); 4,36 (s, 4H, CCH₂O); 5,62-5,65 (m, 2H, C=CH₂) ; 6,12 (s, 2H, C=CH₂).
³¹P-NMR (162 MHz, MeOH-d₄): δ = 24,8.
¹³C-NMR (101 MHz, MeOH-d₄): δ = 18,5 (CH₂=CCH₃); 31,4 (dd, ¹J_{CP} = 137,1 Hz, ³J_{CP} = 5,9 Hz, CH₂P); 39,4 (t, ²J_{CP} = 3,0 Hz, CCH₂P); 68,1 (t, ³J_{CP} = 10,1 Hz, OCH₂CCH₂P); 126,5 (C=CH₂); 137,5 (C=CH₂); 168,2 (C=O).
HRMS (*m*/*z*): Ber. für C₁₃H₂₁O₁₀P₂: 399,06; gef.: 399,06 [M - H]⁻.

### Beispiel 2

### Radikalische Lösungspolymerisation des Bisphosphonsäuredimethacrylats DMPBPA

In Schlenkgefäßen wurden homogene Mischungen von jeweils 2,5 mmol des Bisphosphonsäuredimethacrylats aus Beispiel 1 (DMPBPA) bzw. Glycerindimethacrylat (GDMA; Vergleich) und 1 mol-% (bezogen auf das Monomer) 2,2'-Azobis-(2-methylpropionamidin)-dihydrochlorid in 2,5 ml einer Wasser/Ethanol-Mischung (1:2 v/v) hergestellt und mittels Durchleiten von Argon entgast. Danach wurden die Polymerisationsansätze in einem Thermostat auf 65 °C erwärmt. Die Zeit, nach der sich ein dreidimensionales standfestes Gel ausbildete, wurde als Gelzeit bestimmt.

| **Vernetzer** | **Gelzeit (min)** |
|---|---|
| DMPBPA | 1,5 |
| GDMA (Vergleich) | 1,8 |

Die Ergebnisse zeigen, dass das Bisphosphonsäuredimethacrylat DMPBPA eine dem GDMA vergleichbare Vernetzungsreaktivität zeigt.

### Beispiel 3

### Radikalische Photopolymerisation des Bisphosphonsäuredimethacrylates DMPBPA

Die Photopolymerisationsexperimente wurden mittels eines Perkin Elmer Differential-Scanning-Calorimeter DSC-7 durchgeführt, wobei 0,4 Gew.-% Bis-(4-methoxybenzoyl)diethyl-germanium als Photoinitiator eingesetzt wurden. Jeweils 0,8 mg der untersuchten Monomermischung auf Basis von 2-Hydroxyethylmethacrylat (HEMA) und GDMA bzw. DMPBPA wurden in offene Aluminiumpfännchen gegeben. Vor der Photopolymerisation wurde die DSC-Kammer 5 min mit Stickstoff gespült. Die Proben wurden 2 min bei 37 °C mit einer LED-Lampe Bluephase (Ivoclar Vivadent AG) bestrahlt, wobei die einfallende Lichtintensität 40 mW·cm⁻² betrug und jedes Experiment dreimal wiederholt wurde. Die Polymerisationsgeschwindigkeit Rₚ wurde nach folgender Formel berechet: *Rₚ* = Q/ (m·ΔH_{0P}), wobei Q der Wärmefluss pro Sekunde, m die Masse der Monomerprobe und ΔH_{0P} die Polymerisationsenthalpie von Dimethacrylaten (109.7 kJ·mol⁻¹) darstellt. Aus der DSC-Kurve wurde die Zeit tₘₐₓ bis zum Ereichen der maximalen Polymerisationsgeschwindigkeit R_{p,max} bestimmt.

| **Monomermischung (mol/mol)** | **tₘₐₓ(s)** | **R_{p,max}(s⁻¹)** |
|---|---|---|
| HEMA/GDMA (7:3; Vergleich) | 9,0 ± 0,2 | 0,051 ± 0,004 |
| HEMA/DMPBPA/GDMA (5:2:3) | 2,0 ± 0,1 | 0,096 ± 0,005 |

Die Ergebnisse belegen die sehr hohe Photopolymerisationsreaktivität der DMPBPA-haltigen Mischung im Vergleich zur reinen HEMA/GDMA-Mischung.

### Beispiel 4

### Herstellung eines lichthärtenden Adhäsivs auf Basis von DMPBPA aus Beispiel 1

Zur Untersuchung der Dentinhaftung auf Rinderzahndentin wurde ein Adhäsiv mit der in Tabelle 1 angegebenen Zusammensetzung hergestellt:

**Tabelle 1: Zusammensetzung des Adhäsivs (Angaben in Masse-%**

| Komponente | Adhäsiv |
|---|---|
| DMPBPA | 10,9 |
| Glycerindimethacrylat | 9,9 |
| UDMA¹⁾ | 9,9 |
| Bis-GMA²⁾ | 32,7 |
| 2-Hydroxyethylmethacrylat | 14,9 |
| Photoinitiator³ | 1,7 |
| Ethanol (abs.) | 20,0 |

| | |
|---|---|
| ¹⁾ UDMA (Additionsprodukt aus 2-Hydroxyethylmethacrylat und 2,2,4-Trimethylhexamethylendiisocyanat), ²⁾ Bis-GMA (Additionsprodukt aus Methacrylsäure und Bisphenol-A-diglycidylether), ³⁾ Mischung aus Campherchinon (0,3%), 4-Dimethyl-benzoesäureethylester (0,4%) und dem Acylphosphinoxid Lucerin TPO (1,0%) | |

Rinderzähne wurden so in Kunststoffzylinder eingebettet, dass sich das Dentin und der Kunststoff in einer Ebene befanden. Nach 15 s Ätzung mit 37%iger Phosphorsäure wurde gründlich mit Wasser abgespült. Dann wurde mit einem Microbrush eine Schicht Adhäsiv obiger Zusammensetzung aufgepinselt, mit einem Luftgebläse zur Entfernung des Lösungsmittels kurz verblasen und für 40 s mit einer Halogenlampe (Astralis 7, Ivoclar Vivadent) belichtet. Auf die Adhäsivschicht wurde ein Kompositzylinder aus Tetric® Ceram (Ivoclar Vivadent) in zwei Schichten von je 1-2 mm aufpolymerisiert. Anschließend wurde der Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" zu 31,7 MPa bestimmt.

### Vorschrift A

Zu einer Lösung eines Bisphosphonats (3,75 mmol) in wasserfreiem Dichlormethan (15 ml) wurde Trimethylsilylbromid (2,97 ml, 22,5 mmol, 6,0 äq.) zugetropft und die Reaktionsmischung 5 h bei 30 °C gerührt. Anschließend wurde die Mischung mit Methanol (25 ml) versetzt und 30 min bei Raumtemperatur gerührt. Nach Zugabe von 3,5-Di-tert-butyl-4-hydroxytoluol (BHT, 200 ppm) und Galvinoxyl-Radikalen (20 ppm) wurden die flüchtigen Bestandteile im schwachen Vakuum entfernt und der Rückstand bis zur Gewichtskonstanz im Feinvakuum (0,07 mbar) getrocknet.

### Beispiel 5

### Synthese von 2-Methacryloyloxymethyl-1,3-propylen-bisphosphonsäure

### 1. Stufe: 2-Methacryloyloxymethyl-1,3-propylen-bisphosphonsäure-tetraethylester

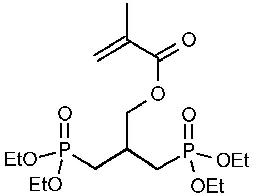

Zu einer Lösung von 2-Hydroxymethyl-1,3-propylen-bisphosphonsäure-tetraethylester (2,24 g, 6,5 mmol), Triethylamin (1,35 ml, 9,7 mmol, 1,5 äq.) und 4-(N,N-Dimethylamino)-pyridin (63 mg, 0,5 mmol, 0,08 äq.) in wasserfreiem Dichlormethan (15 ml) wurde unter Rühren Methacrylsäureanhydrid (1,45 ml, 9,7 mmol, 1,5 äq.) zugegeben und die Reaktionsmischung anschließend 6 h lang unter Rückfluss erhitzt. Danach wurde entionisiertes Wasser (15 ml) zugegeben, die organische Phase abgetrennt und die wässrige Phase mit Dichlormethan (2 x 15 ml) extrahiert. Die vereinigten organischen Phasen wurden mit wasserfreiem Natriumsulfat getrocknet. Nach Entfernung des Lösungsmittels unter vermindertem Druck wurde das erhaltene Rohprodukt durch Säulenchromatographie (Eluent: Ethylacetat/Methanol 91:9) gereinigt. Es wurden 2,0 g (75% Ausbeute) des Produkts als farbloses Öl erhalten.

¹H-NMR (400 MHz, CDCl₃) : δ = 1,31, 1,32 (2t, ³J_{HH} = 7,1 Hz, 12H, POCH₂CH₃) ; 1,90-2,16 (m, 4H, CH₂P); 1,94 (s, 3H, CH₃); 2,53-2,69 (m, 1H, CHCH₂P); 4,04-4,17 (m, 8H, POCH₂CH₃) ; 4,27 (d, ³J_{HH} = 5,1 Hz, 2H, CH₂OCO); 5,56-5,59 (m, 1H, CH₂=C); 6,10 (ls, 1H, CH₂=C).
³¹P-NMR (162 MHz, CDCl₃) : δ = 29,3.
¹³C-NMR (101 MHz, CDCl₃) : δ = 16,4 (d, ³J_{CP} = 6,3 Hz, POCH₂CH₃); 18,4 (s, CH₃); 27,9 (dd, ¹J_{CP} = 141,1 Hz, ³J_{CP} = 10,5 Hz, CH₂P); 28,9 (t, ²J_{CP} = 3,9 Hz, CHCH₂P); 61,7 (d, ²J_{CP} = 7,7 Hz, POCH₂CH₃); 66,9 (t, ³J_{CP} = 9,2 Hz, CH₂OCO); 125,8 (s, CH₂=C); 136,1 (s, CH₂=C); 166, 9 (s, C=O).

### 2. Stufe: 2-Methacryloyloxymethyl-1,3-propylen-bisphosphonsäure

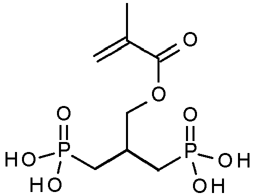

Das Bisphosphonat aus der 1. Stufe (1,53 g, 3,7 mmol) wurde analog zu Vorschrift A umgesetzt. Es wurden 1,12 g (100% Ausbeute) des Produkts als weisse Paste erhalten.

¹H-NMR (400 MHz, D₂O): δ = 1,89 (s, 3H, CH₃); 1,99 (dd, ³J_{HH} = 6,8 Hz; ²J_{HP} = 18,1 Hz, 4H, CH₂P); 2,43-2,58 (m, 1H, CHCH₂P); 4,23 (d, ³J_{HH} = 5,0 Hz, 2H, CH₂OCO); 5,65-5,68 (m, 1H, CH₂=C); 6,11 (ls, 1H, CH₂=C).
³¹P-NMR (162 MHz, D₂O): δ = 29,9.
¹³C-NMR (101 MHz, D₂O): δ = 17,5 (s, CH₃); 28,8 (t, ²J_{CP} = 3,8 Hz, CHCH₂P); 29,3 (dd, ¹J_{CP} = 135,6 Hz, ³J_{CP} = 10,7 Hz, CH₂P); 67,4 (t, ³J_{CP} = 8,8 Hz, CH₂OCO); 127,1 (s, CH₂=C); 135,9 (s, CH₂=C); 169,8 (s, C=O).
HRMS (*m*/*z*): Ber. für C₈H₁₇O₈P₂: 303, 0399; gef.: 303,0403 [M+H]⁺.

### Beispiel 6

### Synthese von 2-(2-Ethoxycarbonyl-propen-3-yloxymethyl)-1,3-propylen-bisphosphonsäure

### 1. Stufe: 2-(2-Ethoxycarbonyl-propen-3-yloxymethyl)-1,3-propylen-bisphosphonsäure-tetraethylester

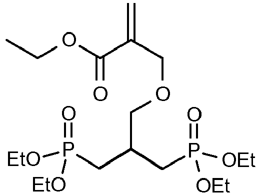

Zu einer Lösung von 2-Hydroxymethyl-1,3-propylen-bisphosphonsäure-tetraethylester (6,0 g, 17,3 mmol) und Triethylamin (3,62 ml, 26 mmol, 1,5 äq.) in wasserfreiem THF (30 ml) wurde Ethyl-2-(chlormethyl)-acrylat (3,86 g, 26,0 mmol, 1,5 äq.) zugegeben und die Reaktionsmischung 45 h bei 70 °C gerührt. Nach Einengen im Vakuum wurde entionisiertes Wasser (50 ml) zugegeben und die erhaltene Lösung mit Ethylacetat (3 x 50 ml) extrahiert. Die vereinigten organischen Phasen wurden mit wasserfreiem Natriumsulfat getrocknet. Nach Entfernung des Lösungsmittels unter vermindertem Druck wurde das erhaltene Rohprodukt durch Säulenchromatographie (Eluent: Ethylacetat/Methanol 98:2) gereinigt. Es wurden 3,77 g (48% Ausbeute) des Produkts als gelbliches Öl erhalten.

¹H-NMR (400 MHz, CDCl₃): δ = 1,28 (t, ³J_{HH} = 7,9 Hz, 3H, COOCH₂CH₃); 1,30 (t, ³J_{HH} = 7,8 Hz, 12H, POCH₂CH₃) ; 1,92-2,10 (m, 4H, CH₂P); 2,37-2,53 (m, 1H, CHCH₂O); 3,60 (d, ³J_{HH} = 5,1 Hz, 2H, CHCH₂O); 4,00-4,15 (m, 8H, POCH₂CH₃) ; 4,17 (s, 2H, OCH₂C); 4,20 (q, ³J_{HH} = 7,1 Hz, 2H, COOCH₂CH₃); 5,81 (s, 1H, CH₂=C); 6,26 (s, 1H, CH₂=C).
³¹P-NMR (162 MHz, CDCl₃) : δ = 30,3.
¹³C-NMR (101 MHz, CDCl₃) : δ = 14,2 (s, COOCH₂CH₃); 16,4 (d, ³J_{CP} = 5,7 Hz, POCH₂CH₃); 27,8 (dd, ¹J_{CP} = 140,2 Hz, ³J_{CP} = 10,7 Hz, CH₂P); 29,7 (t, ²J_{CP} = 3,7 Hz, CHCH₂O); 60,7 (s, COOCH₂CH₃); 61,5 (2d, ²J_{CP} = 6,6 Hz, POCH₂CH₃); 69,1 (s, CH₂=CCH₂O); 73,1 (t, ³J_{CP} = 8,6 Hz, CHCH₂O); 125,4 (s, CH₂=C); 137,4 (s, CH₂=C); 165,8 (s, C=O).

### 2. Stufe: 2-(2-Ethoxycarbonyl-propen-3-yloxymethyl)-1,3-propylen-bisphosphonsäure

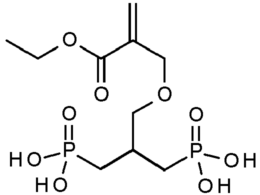

Das Bisphosphonat aus der 1. Stufe (1,69 g, 3,7 mmol) wurde analog zu Vorschrift A umgesetzt. Es wurden 1,28 g (100% Ausbeute) des Produkts als hochviskoses Öl erhalten.

¹H-NMR (400 MHz, D₂O): δ = 1,27 (t, ³J_{HH} = 7,1 Hz, 3H, COOCH₂CH₃); 1,87-2,04 (m, 4H, CH₂P); 2,30-2,45 (m, 1H, CHCH₂O); 3,59 (d, ³J_{HH} = 5,7 Hz, 2H, CHCH₂O); 4,23 (q, ³J_{HH} = 7,1 Hz, 2H, COOCH₂CH₃); 4,25 (s, 2H, OCH₂C) ; 5,94 (s, 1H, CH₂=C); 6,35 (s, 1H, CH₂=C).
³¹P-NMR (162 MHz, D₂O): δ = 28,4.
¹³C-NMR (101 MHz, D₂O): δ = 13,3 (s, COOCH₂CH₃); 28,8 (dd, ¹J_{CP} = 134,5 Hz, ³J_{CP} = 9,6 Hz, CH₂P); 29,1 (t, ²J_{CP} = 2,9 Hz, CHCH₂O); 62,0 (s, COOCH₂CH₃); 69,3 (s, CH₂=CCH₂O) ; 72,6 (t, ³J_{CP} = 9,2 Hz, CHCH₂O); 129,6 (s, CH₂=C); 136,2 (s, CH₂=C); 168,1 (s, C=O).
HRMS (*m*/*z*): Ber. für C₁₀H₂₁O₉P₂: 347, 0661; gef.: 347,0664 [M + H]⁺.

### Beispiel 7

### Synthese von 2- (N-Butyl-acrylamidomethyl)-1,3-propylen-bisphosphonsäure

### 1. Stufe: 2-(Methansulfonyloxymethyl)-1,3-propylen-bisphosphonsäure-tetraethylester

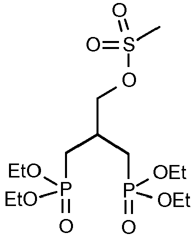

Zu einer Lösung von 2-Hydroxymethyl-1,3-propylen-bisphosphonsäure-tetraethylester (9,0 g, 26,0 mmol) und Triethylamin (4,0 ml, 28,6 mmol, 1,1 äq.) in wasserfreiem Dichlormethan (90 ml) wurde bei 0 °C Methansulfonylchlorid (2,2 ml, 28,6 mmol) zugetropft. Die Reaktionsmischung wurde 30 min bei 0 °C und 2 h bei Raumtemperatur gerührt. Nach Einengen im Vakuum wurde entionisiertes Wasser (100 ml) zugegeben und mit Ethylacetat (3 x 100 ml) extrahiert. Die vereinigten organischen Phasen wurden mit wasserfreiem Natriumsulfat getrocknet. Nach Entfernung des Lösungsmittels unter vermindertem Druck wurde das erhaltene Rohprodukt durch Säulenchromatographie (Eluent: Ethylacetat/Methanol 9:1) gereinigt. Es wurden 9,37 g (85% Ausbeute) des Produkts als farbloses Öl erhalten.

¹H-NMR (400 MHz, CDCl₃) : δ = 1,33 (t, ³J_{HH} = 7,1 Hz, 12H, POCH₂CH₃); 1,89-2,14 (m, 4H, CH₂P); 2,51-2,69 (m, 1H, CHCH₂P); 3,06 (s, 3H, CH₃S); 4,04-4,16 (m, 8H, POCH₂CH₃); 4,42 (d, ³J_{HH} = 4,5 Hz, 2H, CH₂OS).
³¹P-NMR (162 MHz, CDCl₃): δ = 28,4.
¹³C-NMR (101 MHz, CDCl₃) : δ = 16,4 (d, ³J_{CP} = 6,3 Hz, POCH₂CH₃); 27,5 (dd, ¹J_{CP} = 141,0 Hz, ³J_{CP} = 11,3 Hz, CH₂P); 29,3 (t, ²J_{CP} = 3,9 Hz, CHCH₂P); 37,1 (s, CH₃S); 61,8, 61,9 (2d, ²J_{CP} = 6,5 Hz, POCH₂CH₃); 72,1 (t, ³J_{CP} = 8,1 Hz, CH₂OS).

### 2. Stufe: 2-(N-Butyl-aminomethyl)-1,3-propylen-bisphosphonsäure-tetraethylester

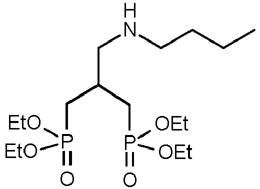

Zum Mesylat aus der 1. Stufe (8,85 g, 20,9 mmol) wurde eine Lösung von Butylamin (20,6 ml, 209 mmol, 10 äq.) in EtOH (110 ml) zugegeben und die Reaktionsmischung 50 h bei 60 °C gerührt. Nach Einengen der Reaktionslösung im Vakuum wurde entionisiertes Wasser (50 ml) zugegeben, mit 10%iger wässriger NaOH auf einen pH-Wert > 11 eingestellt und mit Dichlormethan (3 x 60 ml) extrahiert. Die vereinigten organischen Phasen wurden mit wasserfreiem Natriumsulfat getrocknet und das Lösungsmittel unter vermindertem Druck entfernt. Es wurden 7,0 g (84% Ausbeute) des Produkts als gelbes Öl erhalten und ohne weitere Aufreinigung in der nächsten Stufe eingesetzt.

¹H-NMR (400 MHz, CDCl₃) : δ = 0,90 (t, ³J_{HH} = 7,3 Hz, 3H, CH₂CH₂CH₃); 1,27-1,37 (m, 2H, CH₂); 1,32 (t, ³J_{HH} = 7,0 Hz, 12H, POCH₂CH₃); 1,37-1,48 (m, 2H, CH₂); 1,94-2,10 (m, 4H, CH₂P); 2,22-2,39 (m, 1H, CHCH₂P); 2,57 (t, ³J_{HH} = 7,2 Hz, 2H, CH₂CH₂N); 2,74 (d, ³J_{HH} = 6,1 Hz, 2H, CHCH₂N) ; 4,01-4,17 (m, 8H, POCH₂CH₃) .
³¹P-NMR (162 MHz, CDCl₃) : δ = 30,9.
¹³C-NMR (101 MHz, CDCl₃) : δ = 14,0 (s, CH₂CH₂CH₃); 16,4 (d, ³J_{CP} = 5,4 Hz, POCH₂CH₃); 20,5 (s, CH₂); 28,6 (dd, ¹J_{CP} = 139,0 Hz, ³J_{CP} = 9,6 Hz, CH₂P); 29,5 (t, ²J_{CP} = 3,9 Hz, CHCH₂P); 32,4 (s, CH₂); 49,7 (s, CH₂CH₂N); 53,9 (t, ³J_{CP} = 8,8 Hz, CHCH₂N); 61,4, 61,5 (2d, ²J_{CP} = 6,5 Hz, POCH₂CH₃).

### 3. Stufe: 2-(N-Butyl-acrylamidomethyl)-1,3-propylen-bisphosphonsäure-tetraethylester

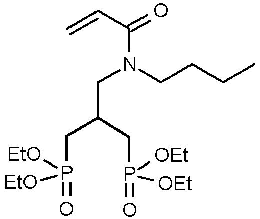

Das Aminophosphonat aus der 2. Stufe (6,93 g, 17,3 mmol) und Triethylamin (2,65 ml, 19,0 mmol, 1,1 äq.) wurden mit trocknem Dichlormethan (40 ml) verdünnt und auf 0 °C abgekühlt. Zu der erhaltenen Mischung wurde eine Lösung von Acrylsäurechlorid (1,41 ml, 17,3 mmol, 1 äq.) in trockenem Dichlormethan (25 ml) zugetropft und die Reaktionsmischung 30 min bei 0 °C und 2 h bei Raumtemperatur gerührt. Anschließend wurde die Reaktionsmischung mit 10%iger wässriger NaOH (2 x 25 ml) gewaschen und die wässrigen Phasen mit Ethylacetat (3 x 50 ml) extrahiert. Die vereinigten organischen Phasen wurden mit wasserfreiem Natriumsulfat getrocknet. Nach Entfernung des Lösungsmittels unter vermindertem Druck wurde das erhaltene Rohprodukt durch Säulenchromatographie (Eluent: Ethylacetat/Methanol 9:1) gereinigt. Es wurden 7,0 g (89% Ausbeute) des Produkts als gelbes Öl erhalten.

¹H-NMR (400 MHz, CDCl₃) : *2 Rotamere* δ = 0,91, 0,93 (2t, ³J_{HH} = 7,2 Hz, 3H, CH₂CH₂CH₃) ; 1,26-1,36 (m, 2H, CH₂); 1,30, 1,31 (2t, ³J_{HH} = 7,0 Hz, 12H, POCH₂CH₃) ; 1,50-1,63 (m, 2H, CH₂); 1,74-1,91 (m, 2H, CH₂P); 1,99-2,15 (m, 2H, CH₂P); 2,38-2,56 (m, 1H, CHCH₂P); 3,33, 3,39 (2t, ³J_{HH} = 7,8 Hz, 2H, CH₂CH₂N); 3,56, 3,64 (2d, ³J_{HH} = 7,5 Hz, 2H, CHCH₂N) ; 4,01-4,16 (m, 8H, POCH₂CH₃) ; 5,66-5,72 (m, 1H, CH₂=CH) ; 6,33, 6,36 (2dd, ²J_{HH} = 2,0 Hz, ³J_{HH} = 16,7 Hz, 1H, CH₂=CH) ; 6,56, 6,67 (2dd, ³J_{HH} = 10,4 Hz, ³J_{HH} = 16,7 Hz, 1H, CH₂=CH).
³¹P-NMR (162 MHz, CDCl₃): 2 *Rotamere* δ = 29,2, 30,2.
¹³C-NMR (101 MHz, CDCl₃) : 2 *Rotamere* δ = 13,8 (2s, CH₂CH₂CH₃); 16,4 (d, ³J_{CP} = 6,6 Hz, POCH₂CH₃); 20,0, 20,3 (2s, CH₂); 27,7 (dd, ¹J_{CP} = 140,3 Hz, ³J_{CP} = 9,1 Hz, CH₂P); 28,3 (t, ²J_{CP} = 3,9 Hz, CHCH₂P); 28,4 (dd, ¹J_{CP} = 139,9 Hz, ³J_{CP} = 9,1 Hz, CH₂P); 29,5 (t, ²J_{CP} = 3,9 Hz, CHCH₂P); 31,5 (s, CH₂); 47,1, 47,7 (2s, CH₂CH₂N); 49,9, 51,9 (2t, ³J_{CP} = 9,2 Hz, CHCH₂N); 61,6, 61,7, 61,8 (4d, ²J_{CP} = 6,6 Hz, POCH₂CH₃); 127,6, 127,8 (2s, CH=CH₂) ; 128,3, 128,4 (2s, CH=CH₂) ; 166,5, 167,0 (2s, C=O).

### 4. Stufe: 2-(N-Butyl-acrylamidomethyl)-1,3-propylen-bisphosphonsäure

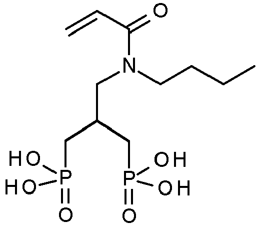

Das Bisphosphonat aus der 3. Stufe (1,69 g, 3,71 mmol) wurde analog zu Vorschrift A umgesetzt. Es wurden 1.26 g (99% Ausbeute) des Produkts als schwach gelber Feststoff erhalten.

¹H-NMR (400 MHz, D₂O): 2 *Rotamere* δ = 0,81 (t, ³J_{HH} = 7,4 Hz, 3H, CH₂CH₂CH₃); 1,16-1,28 (m, 2H, CH₂); 1,42-1,57 (m, 2H, CH₂); 1,68-1,85 (m, 2H, CH₂P); 1,86-2,04 (m, 2H, CH₂P); 2,34-2,54 (m, 1H, CHCH₂P); 3,33, 3,38 (2t, ³J_{HH} = 7,7 Hz, 2H, CH₂CH₂N); 3,52, 3,56 (2d, ³J_{HH} = 7,5 Hz, 2H, CHCH₂N) ; 5,73 (dm, ³J_{HH} = 10,6 Hz, 1H, CH₂=CH) ; 6,11 (dm, ³J_{HH} = 16,9 Hz, 1H, CH₂=CH) ; 6,67, 6,68 (2dd, ³J_{HH} = 10,4 Hz, ³J_{HH} = 16,7 Hz, 1H, CH₂=CH).
³¹P-NMR (162 MHz, D₂O): 2 *Rotamere* δ = 27,6, 28,2.
¹³C-NMR (101 MHz, D₂O): 2 *Rotamere* δ = 12,9, 13,0 (2s, CH₂CH₂CH₃); 19,1, 19,4 (2s, CH₂); 27,5, 28,5 (2t, ²J_{CP} = 3,7 Hz, CHCH₂P); 28,7, 29,2 (2dd, ¹J_{CP} = 135,7 Hz, ³J_{CP} = 9,1 Hz, CH₂P); 30,4 (s, CH₂); 46,7, 47,9 (2s, CH₂CH₂N) ; 50,2, 52,0 (2t, ³J_{CP} = 9,8 Hz, CHCH₂N); 127,4, 127,5 (2s, CH=CH₂) ; 128,9, 129,0 (2s, CH=CH₂); 169, 0, 169, 3 (2s, C=O).
HRMS (*m*/*z*): Ber. für C₁₁H₂₄NO₇P₂: 344, 1028; gef.: 344,1043 [M+H]⁺.

### Beispiel 8

### Herstellung von lichthärtenden Adhäsiven auf Basis der Bisphosphonsäuren aus den Beispielen 5 bis 7

Zur Untersuchung der Dentinhaftung auf Rinderzahndentin wurden Adhäsive mit der in Tabelle 2 angegebenen Zusammensetzung unter Verwendung der Bisphosphonsäuren aus den Beispielen 5 bis 7 hergestellt:

**Tabelle 2: Zusammensetzung der Adhäsive**

| Komponente | Menge (Gew.-%) |
|---|---|
| Bisphosphonsäure gemäß Tabelle 3 | 24,00 |
| DEBAAP¹⁾ | 37,70 |
| Photoinitiator²⁾ | 0,60 |
| BHT³⁾ | 0,03 |
| EtOH | 27,67 |
| Wasser | 10,00 |

| | |
|---|---|
| ¹⁾ N,N'-Diethyl-1,3-bis(acrylamido)propan, ²⁾ Mischung aus Campherchinon (0,6%), ³⁾ 3,5-Di-tert-butyl-4-hydroxytoluol. | |

Frisch extrahierte Rinderzähne wurden so in Kunststoffzylinder eingebettet, dass sich das Dentin bzw. der Schmelz und der Kunststoff in einer Ebene befanden. Die Dentin- bzw. Schmelzoberflächen wurden mit feuchtem Siliciumcarbidpapier (120-grit und 1000-grit) beschliffen. Dann wurde mit einem Microbrush eine Schicht Adhäsiv obiger Zusammensetzung aufgepinselt und 15 s verteilt und die Schicht mit Pressluft getrocknet. Anschliessend wurde mit einem Pinsel AdheSE-Bonding (Ivoclar Vivadent AG) aufgetragen und für 10 s mit einer LED-Lampe (Bluephase G20, Ivoclar Vivadent AG) belichtet. Auf die Adhäsivschicht wurde ein Kompositzylinder aus Tetric® Ceram (Ivoclar Vivadent) in zwei Schichten von je 1-2 mm aufpolymerisiert. Anschließend wurden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" bestimmt. Die Ergebnisse sind in Tabelle 3 aufgeführt und zeigen sehr gute Haftwerte.

**Tabelle 3: Scherhaftfestigkeiten der untersuchten Adhäsive**

| Bisphosphonsäure | Dentin SBS (MPa) | Enamel SBS (MPa) |
|---|---|---|
| Beispiel 5 | 25,1 | 26,0 |
| Beispiel 6 | 17,5 | 27,0 |
| Beispiel 7 | 14,7 | 15,3 |

## Patentansprüche

1. Dentalwerkstoff, der eine polymerisierbare Bisphosphonsäure der Formel III 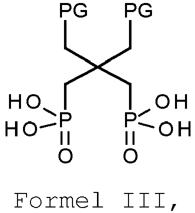 in der
PG unabhängig für eine polymerisierbare Gruppe ausgewählt aus CH₂=CR²-CO-O- und CH₂=CR²-CO-NR³- steht,
R² unabhängig für H oder CH₃ steht und
R³ unabhängig für H oder für einen C₁-C₁₀-Alkyl-Rest steht,
oder der Formel V enthält 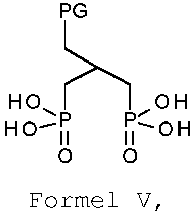 in der
PG für eine polymerisierbare Gruppe ausgewählt aus CH₂=CR²-CO-O-, CH₂=CR²-CO-NR³- und R⁴O-CO-C(=CH₂)-CH₂-O- steht,
R² für H oder CH₃ und vorzugsweise für CH₃ steht,
R³ für H oder für einen C₁-C₁₀-Alkyl-Rest steht und
R⁴ für H, einen C₁-C₁₀-Alkyl-Rest, Phenyl oder Mesityl steht.

2. Dentalwerkstoff nach Anspruch 1, bei dem die polymerisierbare Bisphosphonsäure die Formel III aufweist: 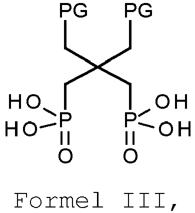 in der
PG unabhängig für eine polymerisierbare Gruppe ausgewählt aus CH₂=CR²-CO-O- und CH₂=CR²-CO-NR³- und insbesondere für CH₂=CR²-CO-O- steht,
R² unabhängig für H oder CH₃ und vorzugsweise für CH₃ steht,
R³ unabhängig für H oder vorzugsweise für einen C₁-C₁₀-Alkyl-Rest, insbesondere für einen C₁-C₇-Rest, bevorzugt für einen C₁-C₅-Rest, weiter bevorzugt für einen C₁-C₃-Rest und am meisten bevorzugt für CH₃ oder C₂H₅ steht.

3. Dentalwerkstoff nach Anspruch 1, bei dem die polymerisierbare Bisphosphonsäure die Formel V aufweist: 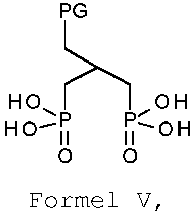 in der
PG für eine polymerisierbare Gruppe ausgewählt aus CH₂=CR²-CO-O-, CH₂=CR²-CO-NR³- und R⁴O-CO-C(=CH₂)-CH₂-O-, insbesondere ausgewählt aus CH₂=CR²-CO-O- und CH₂=CR²-CO-NR³- und bevorzugt für CH₂=CR²-CO-NR³- steht,
R² für H oder CH₃ und vorzugsweise für CH₃ steht,
R³ für H oder vorzugsweise für einen C₁-C₁₀-Alkyl-Rest, insbesondere für einen C₁-C₇-Rest, bevorzugt für einen C₁-C₅-Rest, weiter bevorzugt für einen C₁-C₃-Rest und am meisten bevorzugt für CH₃ oder C₂H₅ steht.

4. Dentalwerkstoff nach Anspruch 3, bei dem
PG für eine polymerisierbare Gruppe ausgewählt aus CH₂=CR²-CO-O- und CH₂=CR²-CO-NR³- und insbesondere für CH₂=CR²-CO-NR³- steht,
R² für H oder CH₃ und vorzugsweise für CH₃ steht,
R³ für H oder vorzugsweise für einen C₁-C₁₀-Alkyl-Rest, insbesondere für einen C₁-C₇-Rest, bevorzugt für einen C₁-C₅-Rest, weiter bevorzugt für einen C₁-C₃-Rest und am meisten bevorzugt für CH₃ oder C₂H₅ steht.

5. Dentalwerkstoff nach einem der Ansprüche 1 bis 4, der ein oder mehrere zusätzliche radikalisch polymerisierbare Monomere enthält.

6. Dentalwerkstoff nach Anspruch 5, der
Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA, UDMA, Di-, Tri- oder Tetra-ethylenglycoldi(meth)acrylat, Trimethylolpropantri(meth)-acrylat, Pentaerythrittetra(meth)acrylat, Glycerindi(meth)-acrylat, 1,4-Butandioldi(meth)acrylat, 1,10-Decandiol-di(meth)acrylat, 1,12-Dodecandioldi(meth)acrylat, und/oder
ein oder mehrere N-mono- oder disubstituierte Acrylamide, N-Ethylacrylamid, N,N-Dimethacrylamid, N-(2-Hydroxyethyl)acrylamid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether, und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acrylamido)-propan, 1,3-Bis(methacrylamido)-propan, 1,4-Bis(acrylamido)-butan, 1,4-Bis(acryloyl)-piperazin, oder eine Mischung davon enthält.

7. Dentalwerkstoff nach einem der Ansprüche 1 bis 6, der ein oder mehrere zusätzliche radikalisch polymerisierbare, säuregruppenhaltige Monomere enthält.

8. Dentalwerkstoff nach Anspruch 7, der
Maleinsäure, Acrylsäure, Methacrylsäure, 2-(Hydroxymethyl)-acrylsäure, 4-(Meth)acryloyloxyethyltrimellitsäureanhydrid, 10-Methacryloyloxydecylmalonsäure, N-(2-Hydroxy-3-meth-acryloyloxypropyl)-N-phenylglycin, 4-Vinylbenzoesäure, und/oder
Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester,
und/oder
2-Methacryloyloxypropylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogen-phosphat, 1,3-Bis-(N-acryloyl-N-propyl-amino)-propan-2-yl-dihydrogenphosphat,
und/oder
Vinylsulfonsäure, 4-Vinylphenylsulfonsäure, 3-(Methacryl-amido)propylsulfonsäure,
oder eine Mischung davon enthält.

9. Dentalwerkstoff nach einem der Ansprüche 1 bis 8, der einen Initiator für die radikalische Polymerisation enthält.

10. Dentalwerkstoff nach einem der Ansprüche 1 bis 9, der organischen und/oder anorganischen Füllstoff enthält.

11. Dentalwerkstoff nach einem der Ansprüche 1 bis 10, der
a) 0,1 bis 50 Gew.-%, insbesondere 1 bis 40 Gew.-%, bevorzugt 2 bis 30 Gew.-% und besonders bevorzugt 5 bis 20 Gew.-% polymerisierbare Bisphosphonsäure der Formel III oder V,
b) 0,01 bis 10 Gew.-%, bevorzugt 0,1 bis 3 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff und
f) 0 bis 70 Gew.-%, bevorzugt 0 bis 60 Gew.-% und besonders bevorzugt 0 bis 50 Gew.-% Lösungsmittel enthält.

12. Dentalwerkstoff nach Anspruch 11 zur Verwendung als Adhäsiv, der 0 bis 20 Gew.-% Füllstoff enthält.

13. Dentalwerkstoff nach Anspruch 11 zur Verwendung als Komposit, der 20 bis 80 Gew.-% Füllstoff enthält.

14. Verwendung einer polymerisierbaren Bisphosphonsäure der Formel III oder V wie in einem der Ansprüche 1 bis 4 definiert zur Herstellung eines Dentalwerkstoffs.

15. Verwendung nach Anspruch 14 zur Herstellung eines Adhäsivs, Zements oder Komposits und insbesondere eines selbstätzenden Adhäsivs oder Zements.

## Claims

1. Dental material that comprises a polymerisable bisphosphonic acid of the Formula III: 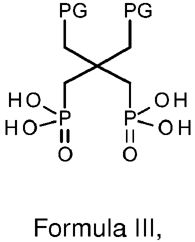 in which
PG independently stands for a polymerisable group selected from CH₂=CR²-CO-O- and CH₂=CR²-CO-NR³-,
R² independently stands for H or CH₃,
R³ independently stands for H or for a C₁-C₁₀ alkyl group,
or of Formula V 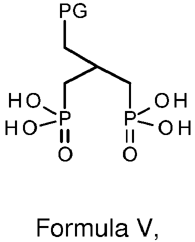 in which
PG stands for a polymerisable group selected from CH₂=CR²-CO-O-, CH₂=CR²-CO-NR³- and R⁴O-CO-C(=CH₂)-CH₂-O
R² stands for H or CH₃ and preferably CH₃,
R³ stands for H or for a C₁-C₁₀ alkyl group, and
R⁴ stands for H, a C₁-C₁₀ alkyl group, phenyl or mesityl.

2. Dental material according to claim 1 in which the polymerisable bisphosphonic acid has the Formula III: 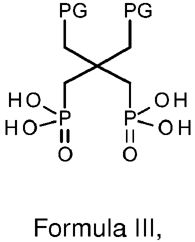 in which
PG independently stands for a polymerisable group selected from CH₂=CR²-CO-O- and CH₂=CR²-CO-NR³-, and in particular for CH₂=CR²-CO-O-,
R² independently stands for H or CH₃ and preferably for CH₃,
R³ independently stands for H or preferably for a C₁-C₁₀ alkyl group, in particular for a C₁-C₇ group, preferably a C₁-C₅ group, further preferably for a C₁-C₃ group and most preferably for CH₃ or C₂H₅.

3. Dental material according to claim 1 in which the polymerisable bisphosphonic acid has the Formula V: 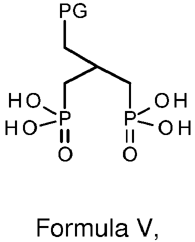 in which
PG stands for a polymerisable group selected from CH₂=CR²-CO-O-, CH₂=CR²-CO-NR³- and R⁴O-CO-C(=CH₂)-CH₂-O, in particular selected from CH₂=CR²-CO-O- and CH₂=CR²-CO-NR³- and preferably CH₂=CR²-CO-NR³-,
R² stands for H or CH₃ and preferably CH₃,
R³ stands for H or for a C₁-C₁₀ alkyl group, in particular for a C₁-C₇ group, preferably a C₁-C₅ group, further preferably for a C₁-C₃ group and most preferably for CH₃ or C₂H₅.
R⁴ stands for H, a C₁-C₁₀ alkyl group, phenyl or mesityl.

4. Dental material according to claim 3 in which
PG stands for a polymerisable group selected from CH₂=CR²-CO-O- and CH₂=CR²-CO-NR³- and in particular CH₂=CR²-CO-NR³-,
R² stands for H or CH₃ and preferably for CH₃,
R³ stands for H or preferably for a C₁-C₁₀ alkyl group, in particular for a C₁-C₇ group, preferably a C₁-C₅ group, further preferably for a C₁-C₃ group and most preferably for CH₃ or C₂H₅.

5. Dental material according to one of claims 1 to 5 which comprises one or more additional radically polymerisable monomers.

6. Dental material according to claim 5 which comprises
methyl, ethyl, hydroxyethyl, butyl, benzyl, tetrahydrofurfuryl or isobornyl (meth)acrylate, bisphenol-A-di(meth)acrylate, bis-GMA, UDMA, di-, tri- or tetraethylene glycol di(meth)acrylate, trimethylolpropane tri(meth)acrylate, pentaerythritol tetra(meth)acrylate, glycerol di(meth)acrylate, 1,4-butanediol di(meth)acrylate, 1,10-decanediol di(meth)acrylate, 1,12-dodecanediol di(meth)acrylate,
and/or
one or more *N*-mono- or -disubstituted acrylamides, *N*-ethylacrylamide, *N*,*N*-dimethacrylamide, *N*-(2-hydroxyethyl)acrylamide, *N*-methyl-*N*-(2-hydroxyethyl)acrylamide, one or more *N*-monosubstituted methacrylamides, *N*-ethylmethacrylamide, *N*-(2-hydroxyethyl)methacrylamide, *N*-vinyl pyrrolidone, one or more crosslinking allyl ethers,
and/or
one or more cross-linking pyrrolidones, 1,6-bis(3-vinyl-2-pyrrolidonyl)-hexane, one or more cross-linking bisacrylamides, methylene- or ethylene-bisacrylamide, one or more cross-linking bis(meth)acrylamides, *N,N'*-diethyl-1,3-bis(acrylamido)-propane, 1,3-bis(methacrylamido)-propane, 1,4-bis(acrylamido)-butane, 1,4-bis(acryloyl)-piperazine,
or a mixture thereof.

7. Dental material according to one of claims 1 to 6 which comprises one or more additional radically polymerisable, acid group-containing monomers.

8. Dental material according to claim 7 which comprises
maleic acid, acrylic acid, methacrylic acid, 2-(hydroxymethyl)acrylic acid, 4-(meth)acryloyloxyethyltrimellitic anhydride, 10-methacryloyloxydecylmalonic acid, *N*-(2-hydroxy-3-methacryloyloxypropyl)-*N*-phenylglycine, 4-vinylbenzoic acid,
and/or
vinylphosphonic acid, 4-vinylphenylphosphonic acid, 4-vinyl-benzylphosphonic acid, 2-methacryloyloxyethylphosphonic acid, 2-methacrylamidoethylphosphonic acid, 4-methacrylamido-4-methyl-pentyl-phosphonic acid, 2-[4-(dihydroxyphosphoryl)-2-oxa-butyl]-acrylic acid, 2-[4-(dihydroxyphosphoryl)-2-oxa-butyl]-acrylic acid ethyl- or -2,4,6-trimethylphenyl ester
and/or
2-methacryloyloxypropyl mono- or dihydrogen phosphate, 2-methacryloyloxyethylphenyl hydrogen phosphate, dipentaerythritol-pentamethacryloyloxyphosphate, 10-methacryloyl-oxydecyl dihydrogen phosphate, phosphoric acid mono-(1-acryloyl-piperidine-4-yl)-ester, 6-(methacrylamido)hexyl dihydrogen phosphate, 1,3-bis-(*N*-acryloyl-*N-*propyl-amino)-propane-2-yl-dihydrogen phosphate,
and/or
vinylsulfonic acid, 4-vinylphenylsulfonic acid, 3-(methacrylamido)propyl sulfonic acid,
or a mixture thereof.

9. Dental material according to one of claims 1 to 8 which comprises an initiator for radical polymerisation.

10. Dental material according to any one of claims 1 to 9 which comprises organic and/or inorganic filler.

11. Dental material according to one of claims 1 to 10 which comprises
a) 0.1 to 50 wt %, in particular 1 to 40 wt %, preferably 2 to 30 wt % and particularly preferably 5 to 20 wt % of polymerisable bisphosphonic acid of Formula III or V,
b) 0.01 to 10 wt %, preferably 0.1 to 3 wt % and particularly preferably 0.2 to 2 wt % initiator,
c) 0 to 80 wt %, preferably 0 to 60 wt % and particularly preferably 5 to 50 wt % comonomer,
d) 0 to 30 wt %, preferably 0.5 to 15 wt % and particularly preferably 1 to 5 wt % adhesive monomer,
e) 0 to 80 wt % filler and
f) 0 to 70 wt %, preferably 0 to 60 wt % and particularly preferably 0 to 50 wt % solvent.

12. Dental material according to claim 11 for use as an adhesive which comprises 0 to 20 wt % filler.

13. Dental material according to claim 11 for use as a composite which comprises 20 to 80 wt % filler.

14. Use of a polymerisable bisphosphonic acid of Formula III or V, as defined in one of claims 1 to 4, for the preparation of a dental material.

15. Use according to claim 14 for the preparation of an adhesive, cement or composite and in particular a self-etching adhesive or cement.

## Revendications

1. Matériau dentaire, qui contient un acide bisphosphonique polymérisable de formule III 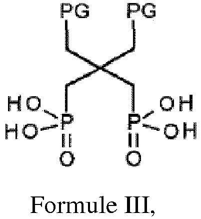 dans laquelle
PG représente chaque fois indépendamment un groupe apte à la polymérisation, choisi parmi les groupes CH₂=CR²-CO-O- et CH₂=CR²-CO-NR³-,
R² représente chaque fois indépendamment un atome d'hydrogène ou un groupe CH₃ et
R³ représente chaque fois indépendamment un atome d'hydrogène ou un radical alkyle en C₁-C₁₀,
ou de formule V 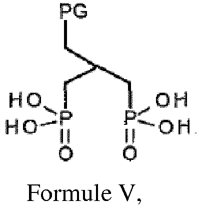 dans laquelle
PG représente un groupe apte à la polymérisation, choisi parmi les groupes CH₂=CR²-CO-O-, CH₂=CR²-CO-NR³- et R⁴O-CO-C(=CH₂)-CH₂-O-,
R² représente un atome d'hydrogène ou un groupe CH₃ et de préférence un groupe CH₃,
R³ représente un atome d'hydrogène ou un radical alkyle en C₁-C₁₀ et
R⁴ représente un atome d'hydrogène, un radical alkyle en C₁-C₁₀, phényle ou mésityle.

2. Matériau dentaire selon la revendication 1, dans lequel l'acide bisphononique polymérisable présente la formule III : 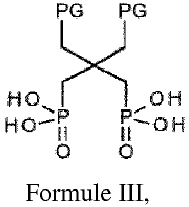 dans laquelle
PG représente chaque fois indépendamment un groupe apte à la polymérisation, choisi parmi les groupes CH₂=CR²-CO-O- et CH₂=CR²-CO-NR³- et en particulier représente le groupe CH₂=CR²-CO-O-,
R² représente chaque fois indépendamment un atome d'hydrogène ou un groupe CH₃ et de préférence un groupe CH₃,
R³ représente chaque fois indépendamment un atome d'hydrogène ou de préférence un radical alkyle en C₁-C₁₀, en particulier un radical en C₁-C₇, de préférence un radical en C₁-C₅, de façon plus particulièrement préférée un radical en C₁-C₃ et de façon tout particulièrement préférée un groupe CH₃ ou C₂H₅.

3. Matériau dentaire selon la revendication 1, dans lequel l'acide bisphononique polymérisable présente la formule V : 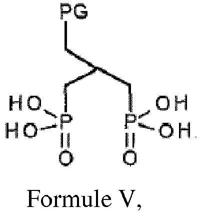 dans laquelle
PG représente un groupe apte à la polymérisation, choisi parmi les groupes CH₂=CR²-CO-O-, CH₂=CR²-CO-NR³- et R⁴O-CO-C(=CH₂)-CH₂-O-, en
R² particulier choisi parmi les groupes CH₂=CR²-CO-O- et CH₂=CR²-CO-NR³- et de préférence représente un groupe CH₂=CR²-CO-NR³-, représente un atome d'hydrogène ou un groupe CH₃ et de préférence un groupe CH₃,
R³ représente un atome d'hydrogène ou de préférence un radical alkyle en C₁-C₁₀, en particulier un radical en C₁-C₇, de préférence un radical en C₁-C₅, de façon plus particulièrement préférée un radical en C₁-C₃ et de façon tout particulièrement préférée un groupe CH₃ ou C₂H₅.

4. Matériau dentaire selon la revendication 3, dans lequel
PG représente un groupe apte à la polymérisation, choisi parmi les groupes CH₂=CR²-CO-O- et CH₂=CR²-CO-NR³-, et en particulier représente un groupe CH₂=CR²-CO-NR³-,
R² représente un atome d'hydrogène et de préférence un groupe CH₃,
R³ représente un atome d'hydrogène ou de préférence un radical alkyle en C₁-C₁₀, en particulier un radical en C₁-C₇, de préférence un radical en C₁-C₅, de façon plus particulièrement préférée un radical en C₁-C₃ et de façon tout particulièrement préférée un groupe CH₃ ou C₂H₅.

5. Matériau dentaire selon l'une quelconque des revendications 1 à 4, qui contient un ou plusieurs monomère(s) supplémentaire(s), polymérisable(s) par voie radicalaire.

6. Matériau dentaire selon la revendication 5, qui contient
du (méth)acrylate de méthyle, éthyle, hydroxyéthyle, butyle, benzyle, tétrahydrofurfuryle ou isobomyle, du di(méth)acrylate de bisphénol A, du bis-GMA, de l'UDMA, du di(méth)acrylate de di-, tri- ou tétraéthylèneglycol, du tri(méth)acrylate de triméthylolpropane, du tétra(méth)acrylate de pentaérythritol, du di(méth)acrylate de glycérol, du di(méth)acrylate de 1,4-butanediol, du di(méth)acrylate de 1,10-décanediol, du di(méth)acrylate de 1,12-dodécanediol,
et/ou
un ou plusieurs acrylamide(s) N-mono- ou disubstitué(s), N-éthylacrylamide, N,N-diméthacrylamide, N-(2-hydroxyéthyl)acrylamide, N-méthyl-N-(2-hydroxyéthyl)-acrylamide, un ou plusieurs méthacrylamide(s) N-monosubstitué(s), N-éthyl-méthacrylamide, N-(2-hydroxyéthyl)méthacrylamide, de la N-vinylpyrrolidone, un ou plusieurs éther(s) allylique(s),
et/ou
une ou plusieurs pyrrolidone(s) réticulante(s), 1,6-bis(3-vinyl-2-pyrrolidonyl)-hexane, un ou plusieurs bisacrylamide(s) réticulant(s), méthylène- ou éthylène-bisacrylamide, un ou plusieurs bis(méth)acrylamide(s) réticulant(s), N,N'-diéthyl-1,3-bis(acrylamido)-propane, 1,3-bis(méthacrylamido)-propane, 1,4-bis(acrylamido)-butane, de la 1,4-bis(acryloyl)-pipérazine, ou un mélange de ceux-ci.

7. Matériau dentaire selon l'une quelconque des revendications 1 à 6, qui contient un ou plusieurs monomère(s) supplémentaire(s), polymérisable(s) par voie radicalaire.

8. Matériau dentaire selon la revendication 7, qui contient
de l'acide maléique, de l'acide acrylique, de l'acide méthacrylique, de l'acide 2-(hydroxyméthyl)-acrylique, de l'anhydride 4-(méth)acryloyloxyéthyltrimellitique, de l'acide 10-méthacryloyloxydécylmalonique, de la N-(2-hydroxy-3-méthacryloyloxypropyl)-N-phénylglycine, de l'acide 4-vinylbenzoïque,
et/ou
de l'acide vinylphosphonique, de l'acide 4-vinylphénylphosphonique, de l'acide 4-vinylbenzylphosphonique, de l'acide 2-méthacryloyloxyéthylphosphonique, de l'acide 2-méthacrylamidoéthylphosphonique, de l'acide 4-méthacrylamido-4-méthyl-pentyl-phosphonique, de l'acide 2-[4-(dihydroxyphosphoryl)-2-oxa-butyl]-acrylique, du 2-[4-(dihydroxyphosphoryl)-2-oxa-butyl]-acrylate d'éthyle ou de 2,4,6-triméthyl-phényle,
et/ou
du mono- ou dihydrogénophosphate de 2-méthacryloyloxypropyle, de l'hydrogénophosphate de 2-méthacryloyloxyéthylphényle, du pentaméthacryloyloxyphosphate de dipentaérythritol, du dihydrogénophosphate de 10-méthacryloyloxy-décyle, du phosphate de mono-(1-acryloylpipéridin-4-yle), du dihydrogénophosphate de 6-(méthacrylamido)hexyle, du dihydrogénophosphate de 1,3-bis-(N-acryloyl-N-propyl-amino)-propan-2-yle,
et/ou
de l'acide vinylsulfonique, de l'acide 4-vinylphénylsulfonique, de l'acide 3-(méthacrylamido)propylsulfonique,
ou un mélange de ceux-ci.

9. Matériau dentaire selon l'une quelconque des revendications 1 à 8, qui contient un amorceur pour la polymérisation radicalaire.

10. Matériau dentaire selon l'une quelconque des revendications 1 à 9, qui contient une charge organique et/ou une charge inorganique.

11. Matériau dentaire selon l'une quelconque des revendications 1 à 10, qui contient
a) 0,1 à 50 % en poids, en particulier 1 à 40 % en poids, de préférence 2 à 30 % en poids et de façon particulièrement préférée 5 à 20 % en poids d'acide bisphosphonique polymérisable de formule III ou V,
b) 0,01 à 10,0 % en poids, de préférence 0,1 à 3 % en poids et de façon particulièrement préférée 0,2 à 2 % en poids d'amorceur,
c) 0 à 80 % en poids, de préférence 1 à 60 % en poids et de façon particulièrement préférée 5 à 50 % en poids de comonomère,
d) 0 à 30 % en poids, de préférence 0,5 à 15 % en poids et de façon particulièrement préférée 1 à 5 % en poids de monomère adhésif,
e) 0 à 80 % en poids de charge et
f) 0 à 70 % en poids, de préférence 0 à 60 % en poids et de façon particulièrement préférée 0 à 50 % en poids de solvant.

12. Matériau dentaire selon la revendication 11, destiné à l'utilisation en tant qu'adhésif, qui contient 0 à 20 % en poids de charge.

13. Matériau dentaire selon la revendication 11, destiné à l'utilisation en tant que composite, qui contient 20 à 80 % en poids de charge.

14. Utilisation d'un acide bisphosphonique polymérisable de formule III ou V tel que défini dans l'une quelconque des revendications 1 à 4, pour la production d'un matériau dentaire.

15. Utilisation selon la revendication 14, pour la production d'un adhésif, ciment ou composite et en particulier d'un adhésif ou ciment automordançant.
